# EUROPÄISCHE PATENTSCHRIFT

(11) **EP 0 912 489 B1**
(45) Veröffentlichungstag und Bekanntmachung des Hinweises auf die Patenterteilung: **13.06.2001**
(21) Anmeldenummer: 97931741.9
(22) Anmeldetag: 02.07.1997
(51) Int. Cl.: C07C 67/307, C07C 69/63

(54) **VERFAHREN ZUR HERSTELLUNG VON 2-FLUORISOBUTTERSÄUREESTERN**
METHOD FOR PRODUCING 2-FLUORO-ISO BUTYRIC ACID ESTERS
PROCEDE POUR FABRIQUER DES ESTERS DE L'ACIDE 2-FLUORO-ISOBUTYRIQUE

(30) Priorität: 05.07.1996 DE 19627150
(43) Veröffentlichungstag der Anmeldung: 06.05.1999
(73) Patentinhaber: Aventis CropScience GmbH, 65929 Frankfurt am Main (DE)
(72) Erfinder: APPEL, Wolfgang, D-65779 Kelkheim (DE); PASENOK, Sergej, D-65779 Kelkheim (DE)
(86) Internationale Anmeldenummer: EP9703473
(87) Internationale Veröffentlichungsnummer: WO9801416

(56) Entgegenhaltungen:
- EP-A- 0 506 059

## Beschreibung

Die vorliegende Erfindung betrifft ein Verfahren zur Herstellung von Estern der 2-Fluorisobuttersäure. Insbesondere betrifft die vorliegende Erfindung ein ökonomisch und ökologisch vorteilhaftes Verfahren zur Herstellung dieser Ester, wobei Fluorwasserstoff oder Flußsäure (HF) als Fluorierungsagens eingesetzt wird.

2-Fluorisobuttersäureester sind von großem Interesse als Zwischenprodukte für die Herstellung technisch wertvoller Triazinherbizide.
Ein solches Triazinherbizid ist z. B. in WO 90/09378 beschrieben:

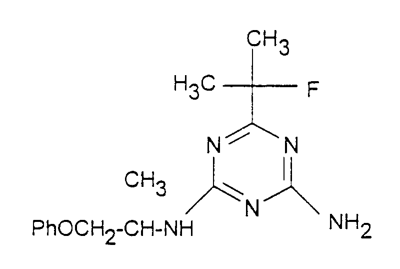

Die Herstellung dieser Triazinherbizide erfolgt z. B. durch Reaktion der entsprechenden Bigunanid-Vorstufe mit 2-Fluorisobuttersäureestern.

Für die Synthese von 2-Fluorisobuttersäureestern sind in der Literatur mehrere Wege beschrieben. Nach J. Org. Chem. 33, 4279 (1968) kann der Fluorisobuttersäureester durch Reaktion des Bromisobuttersäureesters mit AgF hergestellt werden. Die Ausbeuten sind jedoch niedrig, da als Hauptprodukt durch Eliminierung der Methacrylsäureester gebildet wird und die Kosten des AgF machen den Weg ohnehin kommerziell uninteressant.

Nach einem zweiten Verfahren (J. Org. Chem. 55, 3423 (1990)) geht man von Methylisobutyrat aus, das man mit Lithiumdiisopropylamin und Chlortrimethylsilan zum Trimethylsilylether umsetzt, der anschließend mit elementarem Fluor behandelt wird:

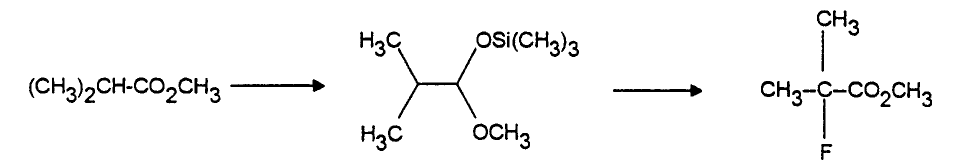

Auch nach diesem Verfahren ist die Ausbeute gering, beide Stufen müssen bei tiefer Temperatur (- 78°C bzw. - 40°C) durchgeführt werden und die Handhabung von elementarem Fluor ist problematisch. Ebenfalls von Silylenolether geht eine Methode aus, die wegen des gefährlichen Fluorierungsagens (Hypofluorit) und der erforderlichen tiefen Temperaturen technisch nicht in Betracht kommt (Du Pont, US 4 215 044).

Nach einem patentierten Verfahren (Idemitsu Kosan, JP 05043515-A) ist die Herstellung von 2-Fluorisobuttersäureestern ausgehend von Methacrylsäureestern und deren Umsetzung mit HF / Amin Reagenzien in Gegenwart von Lewissäuren beschrieben. Die dabei erzielten Ausbeuten liegen allerdings unter 20 %.

In einem weiteren Patent (Idemitsu Kosan, JP 3240-764-A) geht man von Acetoncyanhydrin aus, das zunächst in das entsprechende Methansulfonat überführt wird. Durch Umsetzung mit KF in Diethylenglykol erhält man dann in sehr mäßigen Ausbeuten (12,2 %) das 2-Fluor-2-methylpropionitril.

Ausgehend von α-Hydroxyestern sind in der Literatur mehrere synthetische Methoden beschrieben, die unter Verwendung außerordentlich teurer Reagenzien wie Dimethylaminoschwefeltrifluorid (ICI, EP 468 681), (2-Chloro-1,1,2-trifluoroethyl)- diethylamin (Yarovenko Reagens) (Isr. J. Chem. 8, 925 - 933 (1970)) oder N-Fluorpyridiniumsalzen (Onoda Cement, JP 2 207 228) α-Fluorester liefern. Die Ausbeuten sind allerdings bei α,α-disubstituierten Hydroxyestern wie Hydroxyisobuttersäureester wegen Acrylatbildung nur mäßig und die Verfahren wegen der Art der eingesetzten Reagenzien für technische Verfahren zu teuer.

Zwei neuere patentierte Methoden gehen jeweils von 2-Hydroxyisobuttersäureestern aus. Im ersten Fall (Idemitsu Kosan, EP 506 059 A2 / US 5 175 345) wird das Edukt mit Fluorsulfonsäure ohne oder mit Zusatz einer HF-Quelle umgesetzt, wobei gute Ausbeuten nur bei Zusatz der HF-Quelle (HF / Pyridin: 70 / 30) und nach langen Reaktionszeiten (18 Stunden) erzielt werden.

Im zweiten Fall (Idemitsu Kosan, WO 9424086-A1) wird der 2-Hydroxyisobuttersäureester zunächst mit einem Überschuß Thionylchlorid vorsichtig umgesetzt. Anschließend wird dieses Reaktionsgemisch in einer zweiten Stufe bei tiefer Temperatur (-10°C bis -78°C) in einen großen Überschuß wasserfreier Flußsäure eingetropft. Dieser Schritt muß wegen der heftigen Gasentwicklung (HCI, SOF₂) und der Exothermie sehr langsam durchgeführt werden. Das resultierende Reaktionsgemisch läßt man dann auf Raumtemperatur kommen und arbeitet anschließend wäßrig auf. Bei diesem Verfahren wird im Gegensatz zu den vorher beschriebenen die Menge des als Nebenprodukt entstehenden und schwer abzutrennenden Methacrylats auf einen geringen Prozentsatz reduziert. Das Vorgehen ist allerdings wegen der angesprochenen Probleme wie Gasentwicklung, sehr lange Reaktionszeiten, tiefe Temperaturen für eine Durchführung im industriellen Maßstab sehr aufwendig.

Wegen der erwähnten Bedeutung von 2-Fluorisobuttersäurederivaten für die Synthese bestimmter Triazinherbizide bestand ein Bedürfnis nach einem neuen verbesserten Verfahren für die technische Synthese dieser Verbindungen, das die beschriebenen Nachteile vermeidet und insbesondere ökonomisch und ökologisch vorteilhaft ist.

Erfindungsgemäß wird diese Aufgabe durch ein Verfahren zur Herstellung von 2-Fluorisobuttersäureestern mit der Formel (1) 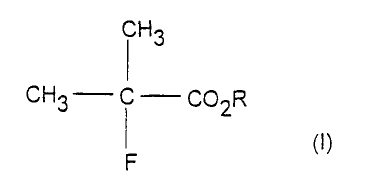 gelöst, wobei R für Methyl, Ethyl, n-Propyl, i-Propyl, n-Butyl, i-Butyl, sec-Butyl und tert.-Butyl oder für Phenyl steht, gemäß dem der entsprechende 2-Hydroxyisobuttersäureester mit Fluorwasserstoff oder Flußsäure als Fluorierungsagens bei einer Temperatur zwischen 0° und 80°C umgesetzt wird.

Wie oben bereits erwähnt gibt es zwei industrielle Verfahren, die von dem auch hier benutzten Edukt ausgehen. Der Austausch der Hydroxylgruppe gegen das Fluoratom wird dabei durch Reaktion mit Fluorsulfonsäure in Gegenwart eines HF / Amingemisches oder im zweistufigen Verfahren mit Thionylchlorid und HF erreicht.

Überraschenderweise wurde nun gefunden, daß sich bei Wahl der geeigneten Temperatur ebendiese Umsetzung auch einfach mit Flußsäure, vorzugsweise mit wasserfreier HF (Fluorwasserstoff), ohne daß weitere Zusätze erforderlich sind, in kurzer Reaktionszeit durchführen läßt, wobei hohe Ausbeuten erzielt werden. Das erfindungsgemäße Verfahren hat zudem den Vorteil, daß das nur schwierig abzutrennende Nebenprodukt Methacrylsäureester lediglich in sehr geringer Menge anfällt.

Das erfindungsgemäße Verfahren ist zudem ökologisch vorteilhaft: die als Fluorierungsagens und Lösungsmittel eingesetzte HF kann nach der Reaktion durch einfaches Abdestillieren recyclisiert werden, da keine Zusätze notwendig sind.

Zur Durchführung der Reaktion setzt man den 2-Hydroxyisobuttersäureester mit einem 10 - 80fachen molaren Überschuß, vorzugsweise 20 - 70fachen molaren Überschuß, an Flußsäure bei 0 - 80°C, vorzugsweise 20 - 75°C und besonders bevorzugt bei 30 - 60 °C, in einem Autoklaven um. Die Reaktionszeit beträgt üblicherweise 3 - 12 Stunden, vorzugsweise läßt man 4 - 7 Stunden abreagieren.

Wird bei niedrigen Temperaturen, zB bei 30 °C und darunter, gearbeitet, kann eine längere Reaktionszeit zur Erhöhung der Ausbeute zweckmäßig sein.

Die Reaktion verläuft üblicherweise bei autogenem Druck.
Danach kann der Überschuß an Fluorierungsagens einfach abdestilliert werden.

Die Aufarbeitung erfolgt nach üblichen, dem Fachmann geläufigen Methoden, indem beispielsweise der Rückstand auf ein Eis/Wasser-Gemisch gegeben wird und das Produkt mit einem geeigneten Lösungsmittel, z. B. Ethylacetat, Methylenchlorid oder Ether, extrahiert wird. Das Produkt, der 2-Fluorisobuttersäureester, kann dann zweckmäßigerweise durch Destillation gewonnen werden.

Nach dem erfindungsgemäßen Verfahren kann das gewünschte Produkt in einer Stufe in hohen Ausbeuten vorteilhaft hergestellt werden.

Die folgenden Beispiele dienen der Erläuterung des erfindungsgemäßen Verfahrens, ohne es jedoch darauf zu beschränken.

### Beispiele

1) 2-Fluorisobuttersäureethylester
   In ein 300 ml Druckgefäß aus Edelstahl, das im Eisbad vorgekühlt wurde, werden 160 g (8 Mol) wasserfreie HF eingefüllt und anschließend 35 g (0,27 Mol) 2-Hydroxyisobuttersäureethylester zugegeben. Das Reaktionsgefäß wird verschlossen und der Inhalt 5 Stunden auf 50°C erwärmt. Dann wird der Reaktor auf 25°C abgekühlt und über eine Kühlfalle entspannt, wobei die überschüssige HF in die Kühlfalle destilliert. Wenn keine weitere HF mehr übergeht wird das Druckgefäß im Eisbad gekühlt, anschließend geöffnet und das Reaktionsgemisch vorsichtig auf 200 g Eis / Wasser gegeben. Die wäßrige Produktphase wird zweimal mit Methylenchlorid extrahiert, der Extrakt einmal mit Wasser gewaschen und über Natriumsulfat getrocknet. Nach Filtration wird das Methylenchlorid bei Normaldruck abdestilliert und anschließend das Produkt durch Vakuumdestillation bei
   13,3 kPa (100 mmHg) / Sdp. 62°C isoliert. Ausbeute: 31,7 g (89 % der Theorie).
2) 2-Fluorisobuttersäuremethylester
   In einem 200 ml Reaktionsgefäß aus Polytetrafluorethylen werden bei 0°C 100 g (5 Mol) wasserfreie HF und 15 g 2-Hydroxyisobuttersäuremethylester (0,13 Mol) vorgelegt. Das Reaktionsgefäß wird verschlossen und 72 Stunden bei 30°C stehengelassen. Anschließend wird über eine Kühlfalle entspannt, wobei die überschüssige HF in dieser Kühlfalle aufgefangen wird. Nach Kühlen auf O°C wird das Reaktionsgefäß geöffnet und der Inhalt auf 100 g Eis / Wasser gegeben. Es wird zweimal mit Methylenchlorid extrahiert. Die vereinigten Extrakte werden einmal mit Wasser gewaschen, dann über Natriumsulfat getrocknet und filtriert. Aus dem Filtrat wird zunächst bei Normaldruck Methylenchlorid abdestilliert.
   Anschließend wird das Produkt durch Destillation bei 13,3 kPa (100 mmHg) / Siedepunkt 52°C gewonnen. Die Ausbeute beträgt 14,4 g (95 % d. Theorie).

### Beispiele 3 - 10

2-Fluorisobuttersäureethylester (Beispiele 3 - 8) und 2-Fluorisobuttersäuremethylester (Beispiele 9 - 11) wurden analog zu den Beispielen 1 und 2 hergestellt, wobei die Reaktionszeiten und die Reaktionstemperaturen variiert wurden, um den Einfluß dieser Parameter auf die Ausbeuten zu untersuchen.

Die Ergebnisse dieser Beispiele sind zusammen mit den Beispielen 1 und 2 in der Tabelle 1 dargestellt.

| Beispiel Nr. | Edukt A. B | Menge g | HF g | Zeit h | Temp. °C | Umsatz % | Produkt % | Acrylat % |
|---|---|---|---|---|---|---|---|---|
| 1 | A | 35 | 160 | 5 | 50 | 95 | 94 | 6 |
| 2 | B | 15 | 100 | 72 | 30 | 99 | 95 | 5 |
| 3 | A | 5 | 60 | 3,5 | 80 | 100 | 46 | 54 |
| 4 | A | 10 | 70 | 4 | 60 | 97 | 87 | 13 |
| 5 | A | 10 | 110 | 4 | 50 | 89 | 92 | 8 |
| 6 | A | 10 | 80 | 22 | 20 | 23 | 100 | - |
| 7 | A | 10 | 80 | 72 | 20 | 42 | 98 | 2 |
| 8 | A | 10 | 80 | 25 | 30 | 55 | 98 | 2 |
| 9 | B | 10 | 30 | 4 | 50 | 62 | 90 | 10 |
| 10 | B | 10 | 80 | 4 | 50 | 98 | 91 | 9 |
| 11 | B | 10 | 80 | 18 | 30 | 85 | 94 | 6 |
| Edukt A: 2-Hydroxyisobuttersäureethylester | | | | | | | | |
| Edukt B: 2-Hydroxyisobuttersäuremethylester | | | | | | | | |
| "Acrylat": Methylmethacrylat | | | | | | | | |

Aus den in Tabelle 1 aufgeführten Ergebnissen läßt sich der Einfluß der Parameter Temperatur, Reaktionsdauer und Molverhältnis der Reaktanten auf die Umsetzung deutlich ablesen. Durch geeignete Wahl dieser Parameter kann die Reaktion gezielt in Richtung Umsatz und/oder Selektivität gesteuert werden.

## Patentansprüche

1. Verfahren zur Herstellung von 2-Fluorisobuttersäureestern der Formel (1) 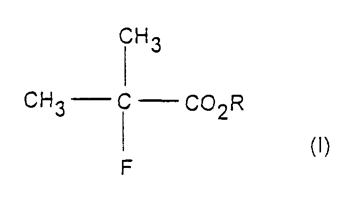 wobei R für Methyl, Ethyl, n-Propyl, i-Propyl, n-Butyl, i-Butyl, sec-Butyl und tert.-Butyl oder Phenyl steht, dadurch gekennzeichnet, daß man den entsprechenden 2-Hydroxyisobuttersäureester mit Fluorwasserstoff oder Flußsäure als Fluorierungsagens bei einer Temperatur zwischen 0° und 80°C umsetzt.

2. Verfahren nach Anspruch 1, dadurch gekennzeichnet, daß das molare Verhältnis von Fluorwasserstoff zu 2-Hydroxyisobuttersäureester zwischen 10 und 80 liegt.

3. Verfahren nach Anspruch 2, dadurch gekennzeichnet, daß das molare Verhältnis von Fluorwasserstoff zu 2-Hydroxyisobuttersäureester zwischen 20 und 70 liegt.

4. Verfahren nach einem der Ansprüche 1 - 3, dadurch gekennzeichnet, daß die Reaktion bei einer Temperatur zwischen 20° und 75°C durchgeführt wird.

5. Verfahren nach einem der Ansprüche 1 - 4, dadurch gekennzeichnet, daß das überschüssige Fluorierungsagens nach der Reaktion abdestilliert wird.

## Claims

1. A process for the preparation of 2-fluoroisobutyrates of the formula (I) 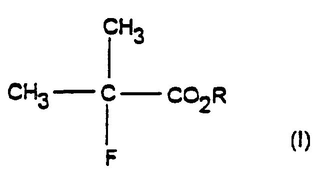 where R is methyl, ethyl, n-propyl, i-propyl, n-butyl, i-butyl, sec-butyl and tert-butyl, or phenyl, which comprises reacting the corresponding 2-hydroxyisobutyrate with hydrogen fluoride or hydrofluoric acid as fluorinating agent at a temperature between 0° and 80°C.

2. The process as claimed in claim 1, wherein the molar ratio of hydrogen fluoride to 2-hydroxyisobutyrate is between 10 and 80.

3. The process as claimed in claim 2, wherein the molar ratio of hydrogen fluoride to 2-hydroxyisobutyrate is between 20 and 70.

4. The process as claimed in one of claims 1 - 3, wherein the reaction is carried out at a temperature between 20° and 75°C.

5. The process as claimed in one of claims 1 - 4, wherein the excess fluorinating agent is distilled off after the reaction.

## Revendications

1. Procédé pour la préparation d'esters de l'acide 2-fluoroisobutyrique de formule (I) 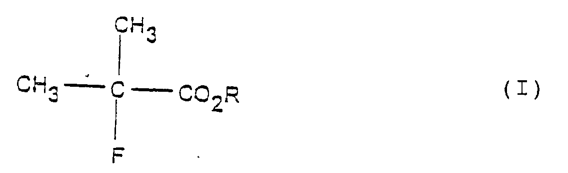 où R représente des groupes méthyle, éthyle, n-propyle, i-propyle, n-butyle, i-butyle, sec-butyle et tert.-butyle ou phényle, caractérisé en ce qu'on fait réagir l'ester de l'acide 2-hydroxyisobutyrique correspondant sur le fluorure d'hydrogène ou l'acide fluorhydrique en tant qu'agent de fluoration, à une température comprise entre 0° et 80°C.

2. Procédé selon la revendication 1, caractérisé en ce que le rapport molaire du fluorure d'hydrogène à l'ester de l'acide 2-hydroxyisobutyrique est compris entre 10 et 80.

3. Procédé selon la revendication 2, caractérisé en ce que le rapport molaire du fluorure d'hydrogène à l'acide 2-hydroxyisobutyrique est compris entre 20 et 70.

4. Procédé selon l'une des revendications 1 à 3, caractérisé en ce qu'on met en oeuvre la réaction à une température comprise entre 20° et 75°C.

5. Procédé selon l'une des revendications 1 à 4, caractérisé en ce qu'on chasse par distillation l'excès en agent de fluoration après la réaction.
